# EUROPEAN PATENT SPECIFICATION

(11) **EP 4 507 622 B1**
(45) Date of publication and mention of the grant of the patent: **27.05.2026**
(21) Application number: 23720184.3
(22) Date of filing: 05.04.2023
(51) Int. Cl.: A61C 7/08, A61F 5/56

(54) **OPTIMIZED MYOFUNCTIONAL DEVICE**
OPTIMIERTE MYOFUNKTIONALE VORRICHTUNG
DISPOSITIF MYO-FONCTIONNEL OPTIMISÉ

(30) Priority: 12.04.2022 IT 202200007223
(43) Date of publication of application: 19.02.2025
(73) Proprietor: Amoruso, Maria, 70132 Bari (IT)
(72) Inventor: Amoruso, Maria, 70132 Bari (IT)
(74) Representative: Conversano, Gabriele
(86) International application number: PCT/IB2023/053484
(87) International publication number: WO 2023/199179

(56) References cited:
- EP-A2- 1 398 061
- WO-A1-2019/144747

## Description

The present invention relates to a myofunctional device, optimized to realize a treatment apt to obtain a correct dental occlusion and a correct lip seal more efficiently than what occurs with the devices known at the state of the art.

### PRIOR ART

Myofunctional orthodontics consists in the reeducation of the lips, cheeks and tongue muscles, as well as in the orthodontic correction by using specific devices.

At the state of the art, there are known many examples of myofunctional devices, realized to treat and solve class II, class III dental crowding. Such devices are also used in children as interceptive orthodontics to guide and correct the bad growth of teeth and maxillary bones, and in adults for the treatment of symptoms as migraine, dizziness, tinnitus, malocclusion, bad posture.

EP 1 398 061 A2 discloses a myofunctional device according to the preamble of claim 1. Technical problem None of the devices known at the state of the art allows the use with teeth aligners; moreover, none of the devices known at the state of the art is optimized to maximize the comfort of usage by users and the efficacy of treatment.

Moreover, none of the known devices is optimized to maximize the efficacy of lip seal correction, or to allow an effective outflow of saliva without needing to remove the device from the oral cavity.

Aim of the invention

Therefore, aim of the present invention is to provide a myofunctional device which can be used together with teeth aligners, and which can be optimized to maximize the comfort of usage by the user and the efficacy of treatment at the same time.

### Brief description of the invention

The device realizes the prefixed aims since it is a myofunctional device (1) comprising an inner arch (12) and an outer arch (13) connected to each other by an occlusal plane (14), said arches (12, 13) and said plane (14) being configured to be introduced in the oral cavity of a patient, so that: the occlusal plane (14) is arranged between the upper dental arch and the lower one; the inner arch (12) is arranged inside the two dental arches, the outer arch (13) is arranged outside the two dental arches, in the space comprised between the same and the inner mucosa of cheeks and lips, characterized in that in the front portion of the outer arch (13) the device (1) comprises a plurality of horizontal reliefs (131), arranged at opposite portions to the middle horizontal plane of the device, in a number of at least two for any portion.

### Description of the figures

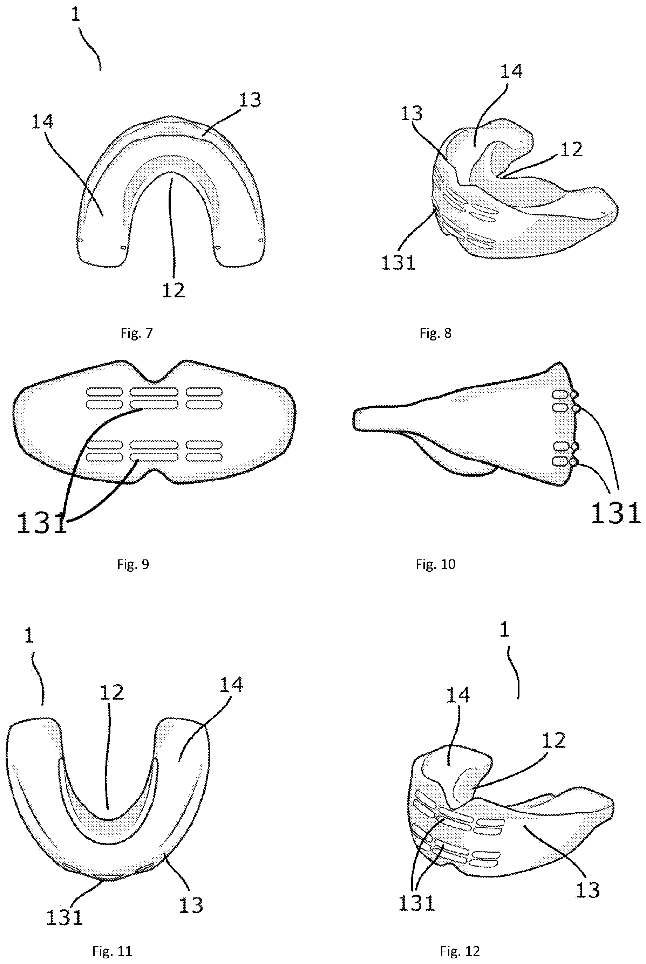

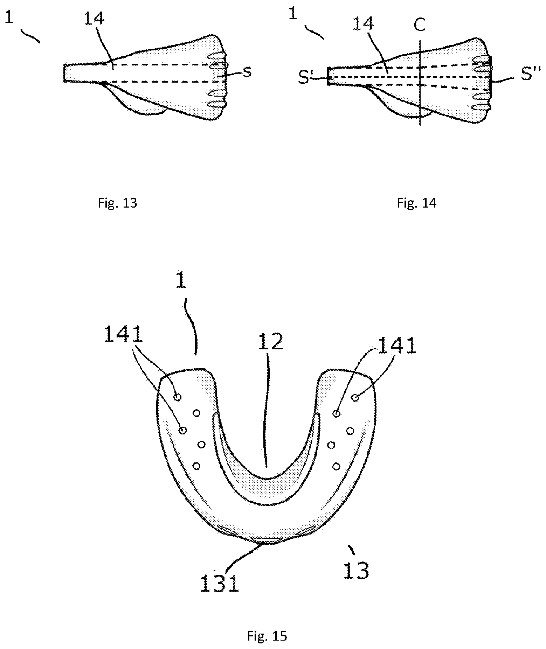

The appended figures 1 to 6 show views from various angles of a first embodiment of the device according to the invention; figures 7 to 12 show views of a second embodiment; figures 13 and 14 show two illustrative views of the thickness variation of the occlusal plane; figure 15 shows an illustrative view of a possible arrangement of the holes for the saliva outflow.

### Description of the invention

With reference to the appended figures, the invention provides a myofunctional device (1) comprising an inner arch (12) and an outer arch (13) connected to each other by an occlusal plane (14), said arches (12, 13) and said plane (14) being configured to be introduced in the oral cavity of a patient, so that:
- the occlusal plane (14) is arranged between the upper dental arch of the patient and the lower one,
- the inner arch (12) is arranged inside the two dental arches of the patient,
- the outer arch is arranged outside the two dental arches of the patient, in the space comprised between the same and the inner mucosa of cheeks and lips.

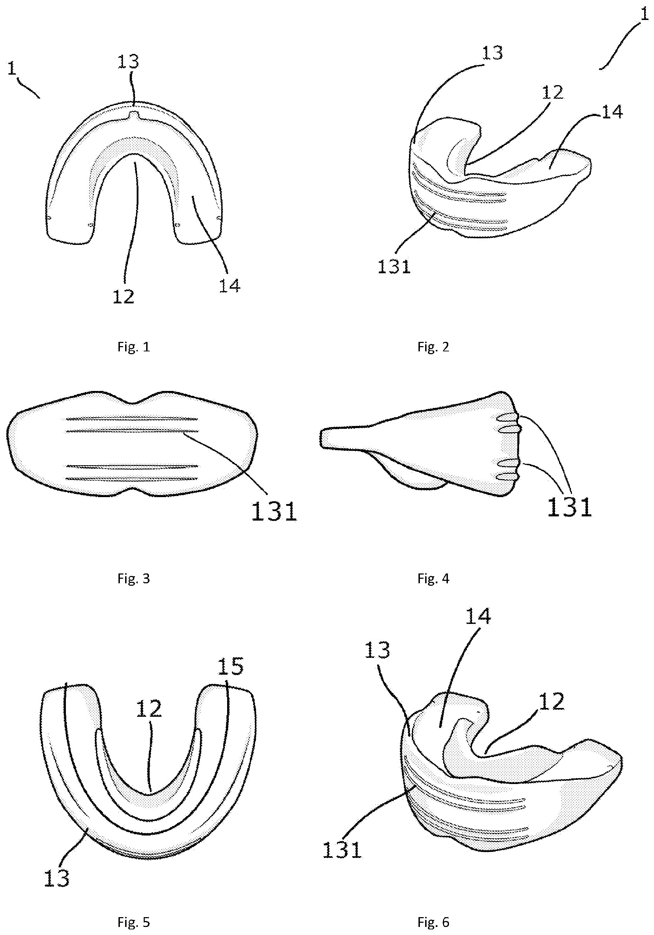

It is to be specified that with inner arch (12) and outer arch (13), as it is shown for example in figure 2, it is intended portions of the device with greater thickness than the occlusal plane (14), positioned respectively inside and outside the same and projecting both above and under the same.

As it is shown in the appended figures, in the front portion of the outer arch (13) the device (1) comprises a plurality of horizontal reliefs (131), arranged at opposite portions to the occlusal (middle) plane of the device, in a number of at least two for any portion.

To such aim it is to be specified that the middle horizontal plane of the device is defined by the occlusal plane (14).

In a first embodiment, such reliefs (131) are shaped as continuous segments; in a second embodiment such reliefs are shaped as discontinuous segments.

The function of the just described reliefs (131) is to allow the patient, both child and adult, to obtain, in a more efficient and natural way, a more precise lip seal than what occurs with the devices known at the state of the art, which are not provided with any relief, or which have round shaped front reliefs or similar.

It is to be specified that with lip seal it is intended the contact between upper lip and lower lip, at rest and without muscle effort. This allows a better physiological deglutition, in addition to a better nasal breathing and a more fluid and comprehensible phonation.

Another feature of the device according to the invention is that the depth (P) of the occlusal plane (14), intended as distance between the inner arch (12) and outer arch (13) is such that it allows the use of the device also by subjects using teeth aligners.

These are devices known at the state of the art with many trade names (ex. Invisalign) which consist in transparent masks worn on teeth with corrective action.

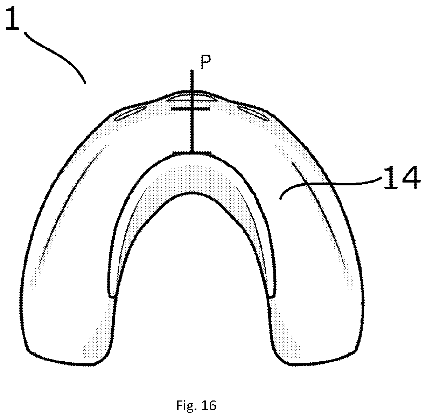

To such aim, the depth of the occlusal plane (14), measured inside the lifted edges defining the outer arch and the inner arch and at the middle of the device, as it is shown in figure 16, is at least 5,5 mm, and preferably greater than 6,5 mm. The shape of the device is also such that the middle is the point where the depth of the occlusal plane (14) is the lowest.

The technical effect of this variation is double. On the one hand, it allows the user of teeth aligners to be able to use also the myofunctional device according to the invention, thus obtaining all the advantages in terms of malocclusion treatment, correction of lip seal, posture etc.

On the other hand, the treatment with teeth aligners becomes more effective and faster. In fact, the presence of the myofunctional device, and the muscle contractions as well as the consequent gingival and dental stimulations increase the blood supply to the periodontal fiber.

Moreover, the presence of the myofunctional device increases also the adherence of the aligners (invisible masks) to teeth, forcing constantly all the dental elements to follow the movements given by the aligners.

Another feature of the device according to the present invention is that preferably the thickness of the occlusal plane (14), measured along the development of the middle arch (15), is not constant.

In a first embodiment, the thickness of the occlusal plane (14) at the front portion of the device is greater than the thickness in the two side portions. In a second embodiment, the thickness of the occlusal plane (14) at the front portion of the device is lower than the thickness in the two side portions.

In a subject without any problems of hypo- or hyper-divergence the occlusal plane (14) of the device has a constant thickness (S), preferably between 4 and 6 mm, and indicatively equal to 5 mm. In a subject with hyper-divergence problems the occlusal plane (14) has a constant thickness (S), lower than the one suitable for subjects without problems, preferably between 2 and 3 mm, and indicatively equal to 2,5 **mm.**

In a subject with hypo-divergence problems, the occlusal plane has a variable thickness, greater in the front portion of the device than in the end portion. In particular, according to a preferred embodiment, the occlusal plane (14) has a constant thickness (S') in the portion at the rear of the position (C) of the upper canine tooth, and an increasing thickness from the canine position to the front portion of the device. Preferably, the constant thickness of the portion at the rear of the position of the upper canine tooth is between 2 and 3 mm, and indicatively is equal to 2,5 mm, and the increase in thickness in the front portion is such that the whole maximum thickness (S") is about 4 mm in the front portion of the device.

Obviously little variations in the dimensions indicated are possible, without departing from the aims of the invention.

Preferably, but not limitingly, the device (1) comprises also a plurality of through holes (141) along the whole development of the occlusal plane (14).

Yet, more preferably, the device comprises a plurality of through holes in the outer portion of the device, having axis inclined to the horizontal, so to allow the saliva to flow therethrough, and so to be swallowed as it occurs physiologically. This makes the use of the device more comfortable, and the patient does not feel the need to remove the device from the mouth to remove and clean the saliva excess formed on the upper and lower plane of the device.

## Claims

1. Myofunctional device (1) comprising an upper and a lower dental arch, an inner arch (12) and an outer arch (13) connected to each other by an occlusal plane (14), said arches (12, 13) and said plane (14) being configured to be introduced in the oral cavity of a patient, so that:
- the occlusal plane (14) is arranged between the upper dental arch of the patient and the lower one;
- the inner arch (12) is arranged inside the two dental arches of the patient,
- the outer arch (13) is arranged outside the two dental arches, in the space comprised between the same and the inner mucosa of cheeks and lips,
**characterized in that**
in the front portion of the outer arch (13) the device (1) comprises a plurality of horizontal reliefs (131), arranged at opposite portions to the middle horizontal plane of the device, which is defined by said occlusal plane (14), in a number of at least two for any portion.

2. Device according to claim 1, **characterized in that** said reliefs (131) are shaped as continuous segments.

3. Device according to claim 1, **characterized in that** said reliefs (131) are shaped as discontinuous segments.

4. Device according to one of the preceding claims, **characterized in that** the depth (P) of said occlusal plane (14), intended as distance between the inner arch (12) and outer arch (13) is such that it allows the use of the device also by subjects using teeth aligners.

5. Device according to claim 4, **characterized in that** the depth of said occlusal plane (14), measured inside said inner arch (12) and said outer arch (13) at the middle of the device, is greater than 5,5 mm, and **in that** the shape of the device is also such that the middle is the point where the depth of the occlusal plane (14) is the lowest one.

6. Device according to one of the preceding claims, **characterized in that** the thickness (S) of said occlusal plane (14), measured along the development of the middle arch (15), is constant.

7. Device according to claim 6, **characterized in that** said thickness (S) is between 4 and 6 mm.

8. Device according to one of claims 1 to 5, **characterized in that** the thickness of said occlusal plane (14) measured along the development of the middle arch (15) is greater in the front portion of the device than in the end portion.

9. Device according to claim 8, **characterized in that** said occlusal plane (14) has a constant thickness (S') in the portion at the rear of the position (C) of the upper canine tooth, and an increasing thickness from the canine position to the front portion of the device.

10. Device according to one of the preceding claims, comprising a plurality of through holes (141) arranged along the development of said occlusal plane (14).

## Patentansprüche

1. Myofunktionales Gerät (1), umfassend einen oberen und einen unteren Zahnbogen, einen inneren Bogen (12) und einen äußeren Bogen (13), die durch eine Okklusionsebene (14) miteinander verbunden sind, wobei die genannten Bögen (12, 13) und die genannte Ebene (14) so konfiguriert sind, dass sie in die Mundhöhle eines Patienten eingeführt werden können, sodass:
- die Okklusionsebene (14) zwischen dem oberen Zahnbogen des Patienten und dem unteren angeordnet ist;
- der innere Bogen (12) innerhalb der beiden Zahnbögen des Patienten angeordnet ist;
- der äußere Bogen (13) außerhalb der beiden Zahnbögen angeordnet ist, im Raum zwischen diesen und der inneren Mukosa der Wangen und Lippen,
**gekennzeichnet dadurch,**
**dass** im vorderen Bereich des äußeren Bogens (13) das Gerät (1) eine Vielzahl von horizontalen Erhebungen (131) aufweist, die an gegenüberliegenden Bereichen zur mittleren Horizontalebene des Geräts, die durch die genannte Okklusionsebene (14) definiert ist, angeordnet sind, in einer Anzahl von mindestens zwei pro Bereich.

2. Gerät nach Anspruch 1, **dadurch gekennzeichnet, dass** die genannten Erhebungen (131) als durchgehende Segmente geformt sind.

3. Gerät nach Anspruch 1, **dadurch gekennzeichnet, dass** die genannten Erhebungen (131) als unterbrochene Segmente geformt sind.

4. Gerät nach einem der vorangegangenen Ansprüche, **dadurch gekennzeichnet, dass** die Tiefe (P) der genannten Okklusionsebene (14), verstanden als Abstand zwischen dem inneren Bogen (12) und dem äußeren Bogen (13), so bemessen ist, dass die Verwendung des Geräts auch bei Personen möglich ist, die Zahnspangen verwenden.

5. Gerät nach Anspruch 4, **dadurch gekennzeichnet, dass** die Tiefe der genannten Okklusionsebene (14), gemessen innerhalb des inneren Bogens (12) und des äußeren Bogens (13) in der Mitte des Geräts, größer als 5,5 mm ist, und dass die Form des Geräts außerdem so gestaltet ist, dass die Mitte der Punkt ist, an dem die Tiefe der Okklusionsebene (14) am geringsten ist.

6. Gerät nach einem der vorangegangenen Ansprüche, **dadurch gekennzeichnet, dass** die Dicke (S) der genannten Okklusionsebene (14), gemessen entlang der Entwicklung des mittleren Bogens (15), konstant ist.

7. Gerät nach Anspruch 6, **dadurch gekennzeichnet, dass** die genannte Dicke (S) zwischen 4 und 6 mm liegt.

8. Gerät nach einem der Ansprüche 1 bis 5, **dadurch gekennzeichnet, dass** die Dicke der genannten Okklusionsebene (14), gemessen entlang der Entwicklung des mittleren Bogens (15), im vorderen Bereich des Geräts größer ist als im hinteren Bereich.

9. Gerät nach Anspruch 8, **dadurch gekennzeichnet, dass** die genannte Okklusionsebene (14) im hinteren Bereich der Position (C) des oberen Eckzahns eine konstante Dicke (S') aufweist und von der Position des Eckzahns bis zum vorderen Bereich des Geräts eine zunehmende Dicke hat.

10. Gerät nach einem der vorangegangenen Ansprüche, umfassend eine Vielzahl von Durchgangslöchern (141), die entlang der Entwicklung der genannten Okklusionsebene (14) angeordnet sind.

## Revendications

1. Dispositif myofonctionnel (1) comprenant une arcade dentaire supérieure et une arcade dentaire inférieure, une arcade interne (12) et une arcade externe (13) reliées entre elles par un plan occlusal (14), lesdites arcades (12, 13) et ledit plan (14) étant configurés pour être introduits dans la cavité buccale d'un patient, de sorte que:
- le plan occlusal (14) est disposé entre l'arcade dentaire supérieure du patient et l'arcade inférieure ;
- l'arcade interne (12) est disposée à l'intérieur des deux arcades dentaires du patient ;
- l'arcade externe (13) est disposée à l'extérieur des deux arcades dentaires, dans l'espace compris entre celles-ci et la muqueuse interne des joues et des lèvres,
**caractérisé en ce que**
dans la portion antérieure de l'arcade externe (13), le dispositif (1) comprend une pluralité de reliefs horizontaux (131), disposés dans des portions opposées au plan horizontal médian du dispositif, défini par ledit plan occlusal (14), en un nombre d'au moins deux pour chaque portion.

2. Dispositif selon la revendication 1, **caractérisé en ce que** lesdits reliefs (131) sont formés sous forme de segments continus.

3. Dispositif selon la revendication 1, **caractérisé en ce que** lesdits reliefs (131) sont formés sous forme de segments discontinus.

4. Dispositif selon l'une des revendications précédentes, **caractérisé en ce que** la profondeur (P) dudit plan occlusal (14), entendue comme la distance entre l'arcade interne (12) et l'arcade externe (13), est telle qu'elle permet l'utilisation du dispositif également par des sujets utilisant des aligneurs dentaires.

5. Dispositif selon la revendication 4, **caractérisé en ce que** la profondeur dudit plan occlusal (14), mesurée à l'intérieur de ladite arcade interne (12) et de ladite arcade externe (13) au milieu du dispositif, est supérieure à 5,5 mm, et **en ce que** la forme du dispositif est également telle que le milieu soit le point où la profondeur du plan occlusal (14) est la plus faible.

6. Dispositif selon l'une des revendications précédentes, **caractérisé en ce que** l'épaisseur (S) dudit plan occlusal (14), mesurée le long du développement de l'arcade médiane (15), est constante.

7. Dispositif selon la revendication 6, **caractérisé en ce que** ladite épaisseur (S) est comprise entre 4 et 6 mm.

8. Dispositif selon l'une des revendications 1 à 5, **caractérisé en ce que** l'épaisseur dudit plan occlusal (14) mesurée le long du développement de l'arcade médiane (15) est plus grande dans la portion antérieure du dispositif que dans la portion postérieure.

9. Dispositif selon la revendication 8, **caractérisé en ce que** ledit plan occlusal (14) présente une épaisseur constante (S') dans la portion postérieure de la position (C) de la canine supérieure, et une épaisseur croissante de la position de la canine vers la portion antérieure du dispositif.

10. Dispositif selon l'une des revendications précédentes, comprenant une pluralité de trous traversants (141) disposés le long du développement dudit plan occlusal (14).
